# EUROPEAN PATENT APPLICATION

(11) **EP 2 939 605 A1**
(43) Date of publication of application: **04.11.2015**
(21) Application number: 14199642.1
(22) Date of filing: 22.12.2014
(51) Int. Cl.: A61B 17/064

(54) **SURGICAL FASTENER HAVING A CAP**

(30) Priority: 28.04.2014 US 201461985060 P; 17.10.2014 US 201414516628
(71) Applicant: Covidien LP, Mansfield, MA 02048 (US)
(72) Inventor: Thomas, Jonathan, New Haven, CT Connecticut 06511 (US)
(74) Representative: Soames, Candida Jane

(57) **Abstract**

A surgical fastener for attaching a prosthesis to tissue including a cap and a coil body. The cap defines a proximal surface and a bore. The coil body extends distally from the cap. The coil body defines a channel configured to receive a drive rod of a surgical applicator inserted through the bore of the cap.

## Description

### CROSS-REFERENCE TO RELATED APPLICATIONS

This application claims the benefit of and priority to U.S. Provisional Patent Application No. 61/985,060, filed April 28, 2014, the entire disclosure of which is incorporated by reference herein.

### BACKGROUND

### Technical Field

The present disclosure relates to a surgical device and, more particularly, to a surgical fastener for attaching a prosthesis in place in the repair of a defect in tissue such as an inguinal hernia.

### Background of Related Art

During hernia repair surgery it is often necessary to affix a section of a mesh over herniated tissue. This is often accomplished through the use of surgical fasteners such as, e.g., staples, sutures, or other affixation type devices.

One method of affixing a mesh to tissue is through the use of surgical fasteners such as screws, tacks, and coils. However, known coils include a traumatic proximal end that may unnecessarily injure tissue if the coil is being rotated too forcefully. Furthermore, many of these coils are made of non-biodegradable material such as stainless steel and are not configured to be removed after they have been implanted in the patient.

Thus, it would be desirable to have a surgical fastener that has an atraumatic proximal end that protects mesh, tissue, and organs from damage.

### SUMMARY

In accordance with an embodiment of the present disclosure, there is provided a surgical fastener for attaching a prosthesis to tissue. The surgical fastener includes a cap and a coil body. The cap defines a proximal surface and a bore. The coil body extends distally from the cap. The coil body defines a channel configured to receive a drive rod of a surgical applicator inserted through the bore of the cap.

In an embodiment, the bore of the cap may have a non-circular cross-section. The bore may include a planar portion and an arcuate portion.

In an embodiment, the coil body may be a continuous double helical coil having a distal end and a proximal end. The distal end may terminate with dual tips. Each tip may be configured to pierce through tissue.

In an embodiment, the cap may be over-molded onto a portion of the coil body. In particular, the cap may be over-molded onto a proximal portion of the coil body. In another embodiment, a diameter of the cap may be larger than a diameter of the coil body. In addition, the coil body may have a uniform diameter along a length thereof.

In an embodiment, the cap may include at least one of glycerol or collagen. In addition, the cap may be formed of a biologic material. The cap may be formed of polymer or copolymer material. The cap may be formed of absorbable, non-absorbable, or partially absorbable polymer or copolymer material.

In an embodiment, the coil body may be longitudinally collapsible and expandable.

In an embodiment, the cap may have a drive thread formed on a peripheral portion thereof. In particular, a leading edge of the drive thread may be spaced apart from a proximal-most coil of the coil body to define a discontinuity therebetween. In addition, a trailing edge of the drive thread may include a proximal surface disposed flush with the proximal surface of the cap.

In an embodiment, the coil body may be formed from a biocompatible metal.

In an embodiment, the coil body may include a tip configured to penetrate tissue as the surgical fastener is rotated relative to tissue.

### BRIEF DESCRIPTION OF THE DRAWINGS

Various embodiments of the present disclosure are described hereinbelow with reference to the drawings, wherein:
FIG. 1 is a side view of a surgical applicator for use with a surgical fastener in accordance with an embodiment of the present disclosure;
FIG. 2a is a perspective view of a prior art coil fastener;
FIG. 2b is a top view of another prior art coil fastener;
FIG. 3 is a perspective view of the surgical fastener in accordance with an embodiment of the present disclosure;
FIG. 4 is a side view of the surgical fastener of FIG. 3;
FIG. 5 is a bottom end view of the surgical fastener of FIG. 3;
FIG. 6a is a side view of a drive rod of the surgical applicator of FIG. 1;
FIG. 6b is a cross-sectional view of the drive rod of FIG. 6a;
FIG. 7 is a top view of a hernia mesh with a plurality of surgical fasteners of FIG. 2 affixed thereto;
FIG. 8 is a perspective view of a surgical fastener in accordance with another embodiment of the present disclosure;
FIG. 9 is a side view of the surgical fastener of FIG. 8;
FIG. 10 is a bottom end view of the surgical fastener of FIG. 8;
FIG. 11 is a side view of a surgical fastener in accordance with yet another embodiment of the present disclosure; and
FIG. 12 is a side view of a surgical fastener in accordance with still yet another embodiment of the present disclosure.

### DETAILED DESCRIPTION

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the present disclosure in unnecessary detail.

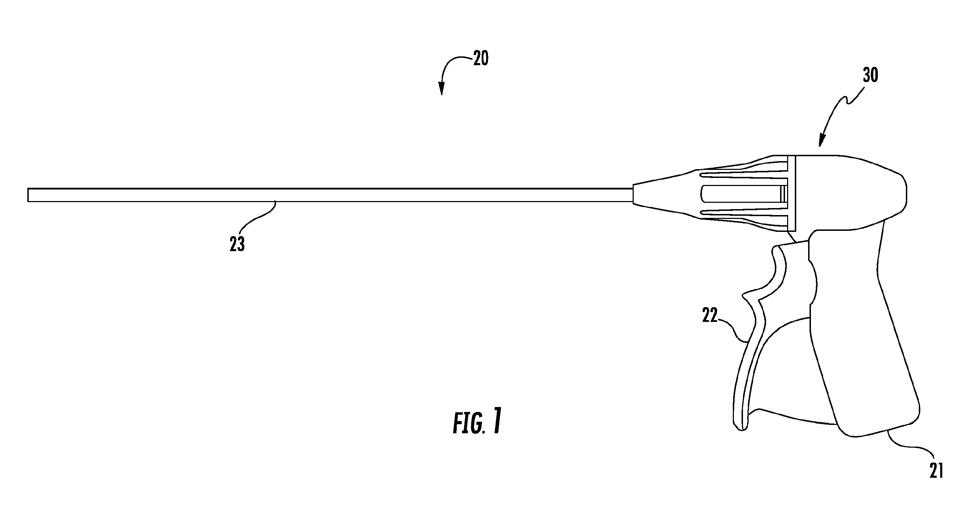

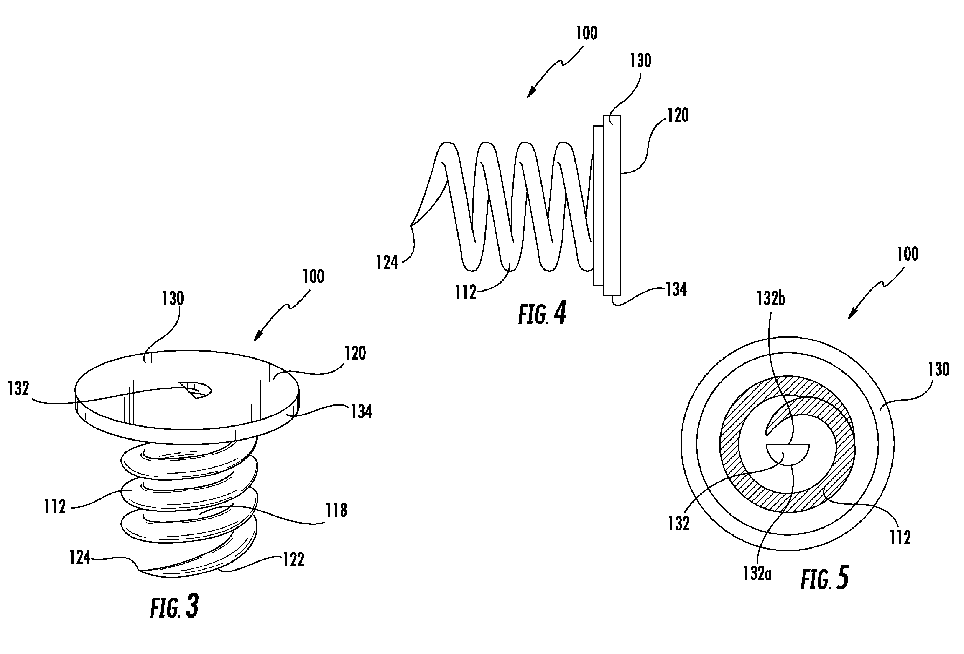

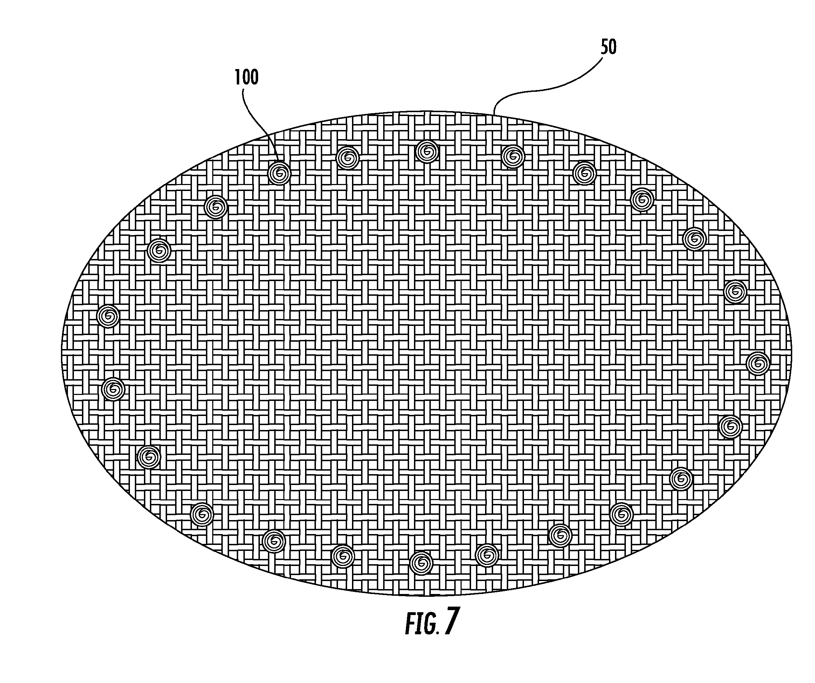

With reference to FIG. 1, there is illustrated a surgical applicator 20 for use with a surgical fastener 100 (FIG. 3) suitable for insertion through a hernia mesh 50 (FIG. 7) and tissue. Applicator 20 generally includes a handle assembly 30 including a pistol grip handle 21 and an actuator 22 and an outer tube 23 extending distally from handle assembly 30. Outer tube 23 contains a plurality of serially arranged surgical fasteners 100. Actuation of actuator 22 ejects each surgical fastener 100 out of outer tube 23 in sequence and into mesh 50 and body tissue. An outer diameter of outer tube 23 is approximately 5 mm for use with standard trocars or laparoscopic devices for minimally invasive entry into the abdomen. Reference may be made to U.S. Patent Nos. 7,867,252 and 8,282,670, the entire contents of each of which are incorporated herein by reference, for a more detailed discussion of the structure and operation of applicator 20.

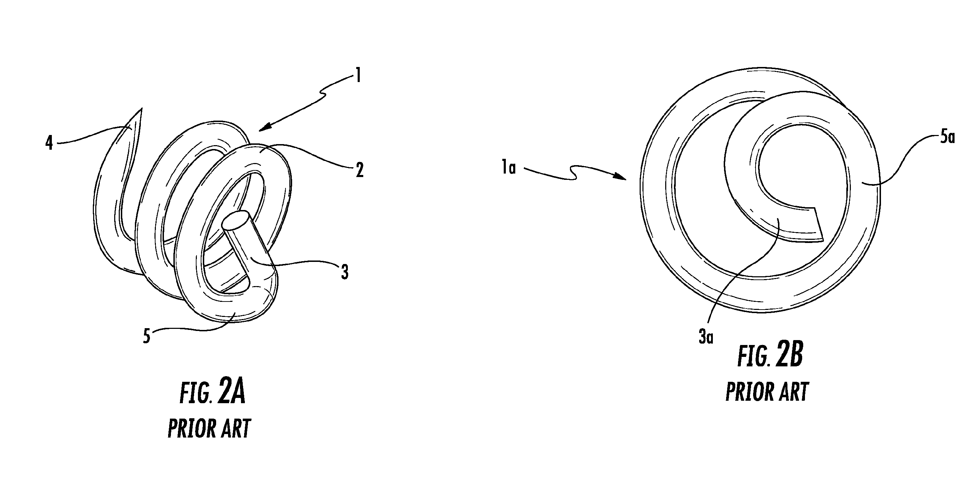

With brief reference to FIG. 2a, there is illustrated a prior art coil fastener 1. Coil fastener 1 is designed to be applied to tissue by rotating the coil into and through tissue. Coil 1 generally includes a coil body portion 2 terminating in a sharp tissue penetrating point 4. A tang 3 is provided at a proximal end 5 of coil body portion 2. Tang 3 extends generally inwardly toward the center of coil body portion 2. Alternatively, a coil fastener 1a may include a proximal end 5a formed into a spiral 3a, as shown in FIG. 2b.

With reference now to FIGS. 3-5, there is illustrated a surgical fastener 100 in accordance with an embodiment of the present disclosure. Surgical fastener 100 includes a cap 130 and a helical coil 112 extending distally from cap 130. Cap 130 may be over-molded onto helical coil 112 such that a proximal end (not shown) of helical coil 112 is enclosed by cap 130. Under such a configuration, cap 130 inhibits the traumatic proximal end of helical coil 112 from contacting and/or injuring tissue and organs, e.g., viscera, to inhibit, e.g., visceral adhesions.

Cap 130 has a larger diameter than a diameter of helical coil 112. Under such a configuration, cap 130 operatively engages an inner surface (not shown) of outer tube 23 (FIG. 1) of applicator 20 such that helical coil 112 is free of contact with the inner surface of outer tube 23, as will be discussed hereinbelow. Cap 130 includes a flat proximal surface 120 configured to reduce visceral attachment and adhesion. In addition, proximal surface 120 may include a predetermined roughness and structure to further reduce visceral adhesions. Moreover, cap 130 may include materials known to reduce visceral adhesions such as, e.g., glycerol and collagen. In addition, cap 130 may be made with a hydrogel network that would swell in physiological fluid, thereby creating a water barrier layer on the surface of cap 130. In addition, cap 130 may be used to deliver therapeutic agents to the surgical site, e.g., an extended release of analgesic or paralytics to address pain. Moreover, cap 130 may be formed of a biologic material, polymer, copolymer, or any combination thereof. In particular, the polymer or copolymer used to form cap 130 may be absorbable, non-absorbable, or partially absorbable.

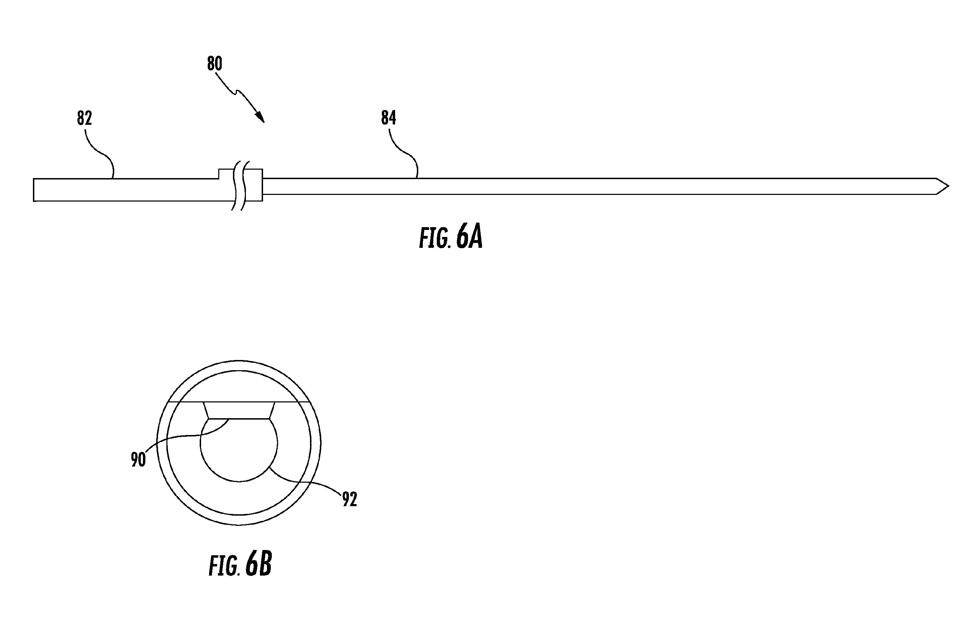

Cap 130 defines a bore 132 configured to receive a drive rod 80 (FIG. 6a) of applicator 20. Drive rod 80 and bore 132 have complementary cross-sections for concomitant rotation of cap 130 and drive rod 80. In particular, bore 132 includes an arcuate portion 132a and a planar portion 132b that define a D-shaped cross-section. In addition, helical coil 112 defines a throughbore 118 (space bounded by coils of helical coil 112) in communication with bore 132. Throughbore 118 is dimensioned to receive drive rod 80 of applicator 20 inserted through bore 132.

With particular reference to FIGS. 3 and 4, the maximum diameter of cap 130 is larger than the maximum diameter of helical coil 112 such that as surgical fastener 100 is rotated within outer tube 23 of applicator 20, a peripheral portion 134 of cap 130 engages the inner thread (not shown) of outer tube 23, while helical coil 112 is free of contact with the inner surface of outer tube 23 which, in turn, inhibits potential damage to helical coil 112.

With continued reference to FIGS. 3 and 4, helical coil 112 extends distally from cap 130. Helical coil 112 defines throughbore 118 therethrough. Helical coil 112 is in a form of a continuous helix that may be longitudinally collapsible and expandable. Helical coil 112 has a uniform diameter along a length thereof. However, helical coil 112 may be tapered along the length thereof to facilitate insertion into tissue. In a particular application such as mesh anchoring for hernia repair, the pre-formed pitch may be about 0.050 inches. However, the pre-formed pitch can vary from 0 to a maximum of approximately 3.0 times the coil diameter. Alternatively, the pitch may vary along the length of helical coil 112 in order to optimize the retaining force of surgical fastener 100. Moreover, since the continuous helical coil 112 is longitudinally collapsible and expandable, upon insertion into tissue, the final pitch may be different than the pre-formed pitch. If helical coil 112 is made of rigid construction, as is also contemplated, the pitch may be made fixed.

Helical coil 112 may be made from semi-stiff implantable wire, such as titanium, wound into a helical shape. Alternatively, helical coil 112 may comprise plastic or absorbable materials. Examples of materials that can be used in constructing helical coil 112 may include titanium, titanium alloys, stainless steel, nickel, chrome alloys and any other biocompatible implantable metals. Alternatively, other options for materials are liquid crystal polymers, HDPE, polyglycolic acid, and polyglycolid hydroxgacetic acid. At least a portion of helical coil 112 may be coated with a biocompatible lubricious material that provides for easier delivery of surgical fastener 100 into tissue.

A distal end 122 of surgical fastener 100 terminates at a tip 124 configured to provide an initial point contact with tissue. Tip 124 may be sharp or blunt depending upon type of tissue to which surgical fastener 100 is affixed. Additionally, one or more barbs or a sharp point (not shown) projecting in reverse direction to tip 124 may be added to helical coil 112 adjacent, e.g., tip 124, to enhance anchoring characteristics of surgical fastener 100.

Referring now to FIGS. 6a and 6b, outer tube 23 (FIG. 1) of applicator 20 includes rotatable drive rod 80 positioned within outer tube 23. Drive rod 80 generally includes a proximal end section 82 configured to operatively engage an actuation mechanism of applicator 20 such that actuation of the actuator 22 rotates drive rod 80 relative to outer tube 23. Drive rod 80 rotates but is axially stationary. Drive rod 80 also includes an elongate section 84 extending distally from proximal end section 82. Elongate section 84 has a sufficient length to receive multiple surgical fasteners 100 therealong. Elongate section 84 is configured and dimensioned to extend through throughbore 118 and bore 132 of surgical fastener 100. In particular, elongate section 84 has a cross-section complementary to a cross-section of bore 132, such that rotation of drive rod 80 causes concomitant rotation of surgical fastener 100. Specifically, elongate section 84 of drive rod 80 includes a flat portion 90 and an arcuate portion 92 that define a generally D-shaped cross-section configured to engage the generally D-shaped bore 132 of surgical fastener 100.

An inner surface (not shown) of outer tube 23 (FIG. 1) includes an inner thread (not shown) which is configured to engage peripheral portion 134 (FIGS. 3 and 4) of cap 130. The inner thread of outer tube 23 may extend completely or partially along the inner surface of outer tube 23. If the inner thread is only provided at the distal end of outer tube 23, a biasing member may be used to bias surgical fasteners 100 distally toward the inner thread at the distal end of outer tube 23. Various known handle mechanisms may be provided to rotate drive rod 80 relative to outer tube 23. Reference may be made to U.S. Patent Nos. 7,867,252 and 8,282,670, the entire contents of each of which are incorporated herein by reference, for a more detailed discussion of the handle mechanism of applicator 20.

In use, applicator 20 containing surgical fastener 100 is positioned against mesh 50 (FIG. 7) covering the desired surgical site, e.g., the hernial defect. Thereafter, actuator 22 is actuated to cause rotation of drive rod 80 relative to outer tube 23 which, in turn, causes peripheral portion 134 of cap 130 of surgical fastener 100 to engage the inner thread (not shown) of outer tube 23 and drive surgical fastener 100 through mesh 50 and into tissue. Moreover, surgical fastener 100 may be removed from mesh 50 and tissue by threadably engaging cap 130 with the inner thread of outer tube 23 of applicator 20 and rotating drive rod 80 in a reverse direction.

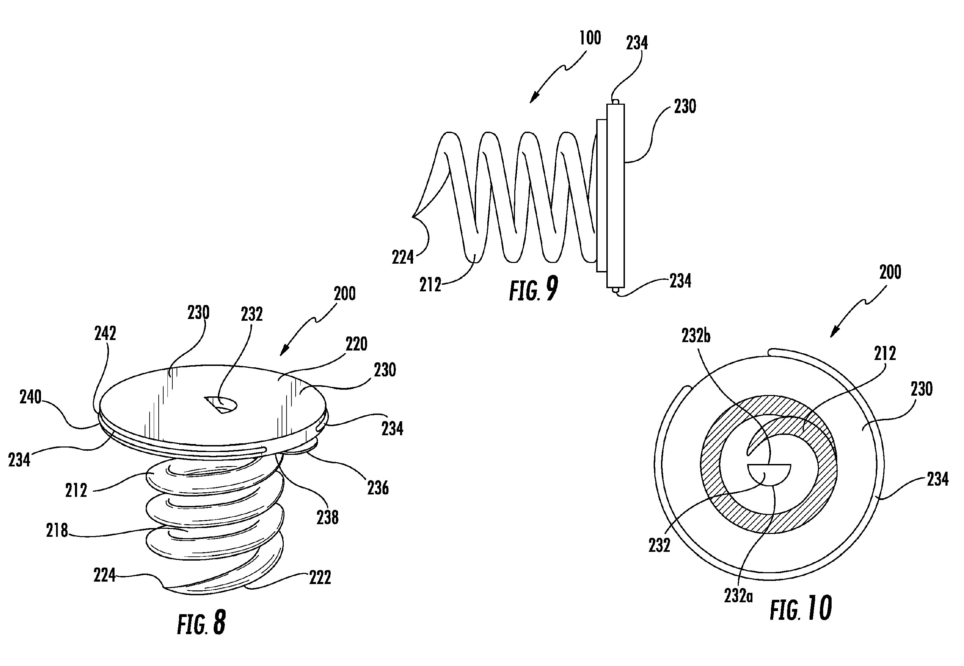

With reference now to FIGS. 8-10, there is illustrated a surgical fastener 200 in accordance with another embodiment of the present disclosure. Surgical fastener 200 is substantially identical to surgical fastener 100. Surgical fastener 200 includes a helical coil 212 and a cap 230 defining a planar proximal surface 220 and a bore 232 configured to receive drive rod 80 (FIG. 6a) of applicator 20 therethrough. Cap 230 may be over-molded onto helical coil 212 such that a proximal end (not shown) of helical coil 212 is enclosed by cap 230. Cap 230 of surgical fastener 200 has a circular cross-section. Drive rod 80 and bore 232 have complementary cross-sections for concomitant rotation of cap 230 and drive rod 80. In particular, bore 232 includes an arcuate portion 232a and a planar portion 232b that define a D-shaped cross-section. In addition, helical coil 212 defines a throughbore 218 (space bounded by coils of helical coil 212) in communication with bore 232. Throughbore 218 is dimensioned to receive drive rod 80 of applicator 20 inserted through bore 232.

However, in contrast to surgical fastener 100, cap 230 is further provided with a drive thread 234 on a peripheral portion of cap 230. Drive thread 234 has a leading edge 236 at a distal end 238 of drive thread 234 and a trailing edge 240 at a proximal end 242 of drive thread 234. The maximum diameter of drive thread 234 is larger than the maximum diameter of helical coil 212 such that as surgical fastener 200 is rotated within outer tube 23 of applicator 20, drive thread 234 engages the inner thread (not shown) of outer tube 23, while helical coil 212 is free of contact with the inner surface of outer tube 23 which, in turn, inhibits potential damage to helical coil 212.

With continued reference to FIGS. 8 and 9, leading edge 236 and trailing edge 240 of drive thread 234 are chamfered to facilitate insertion into applicator 20. Further, trailing edge 240 is flush with proximal surface 220 of cap 230 to facilitate reengagement of surgical fastener 200 by applicator 20 to facilitate removal of surgical fastener 200.

Helical coil 212 extends distally from cap 230. However, a proximal-most coil of helical coil 212 and leading edge 236 of drive thread 234 are discontinuous and are spaced apart, thereby defining a discontinuity. Helical coil 212 is in a form of a continuous helix that may be longitudinally collapsible and expandable. A distal end 222 of surgical fastener 200 terminates at a tip 224 configured to provide an initial point contact with tissue.

As with surgical fastener 100, the configuration of surgical fastener 200, e.g., the pre-formed pitch and diameter, may be varied for different applications and a barb (not shown) may be employed to enhance anchoring in tissue. Moreover, the materials contemplated are the same as those for helical fastener 100.

In use, applicator 20 containing surgical fastener 200 is positioned against mesh 50 (FIG. 7) covering the desired surgical site, e.g., the hernial defect. Thereafter, actuator 22 is actuated to cause rotation of drive rod 80 relative to outer tube 23 which, in turn, causes drive thread 234 of cap 230 of surgical fastener 200 to engage the inner thread (not shown) of outer tube 23 and drive surgical fastener 200 through mesh 50 and into tissue. Moreover, surgical fastener 200 may be removed from mesh 50 and tissue by threadably engaging drive thread 234 of cap 230 with the inner thread of outer tube 23 of applicator 20 and rotating drive rod 80 in a reverse direction.

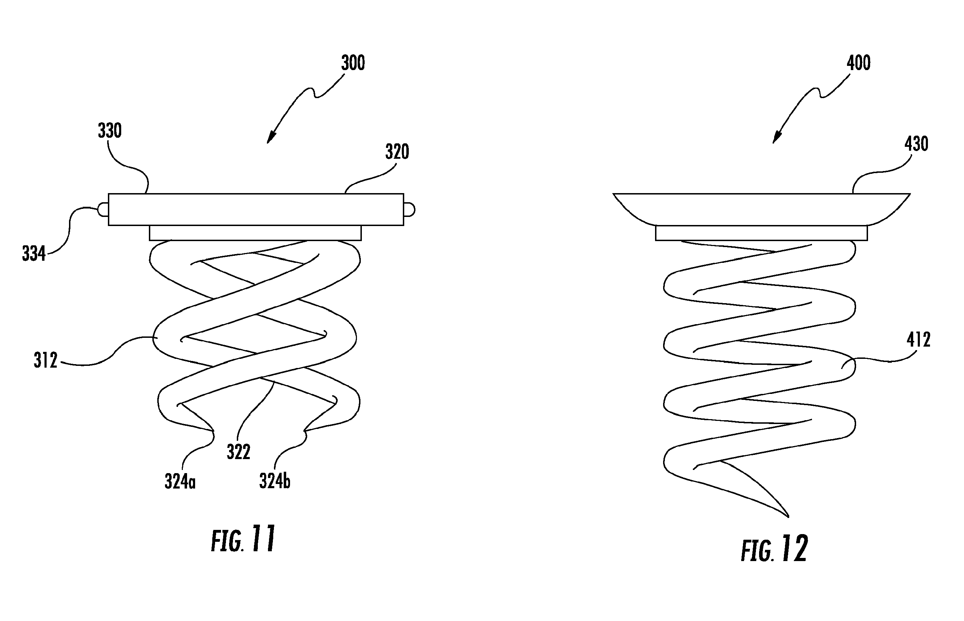

With reference now to FIG. 11, there is illustrated a surgical fastener 300 in accordance with another embodiment of the present disclosure. Surgical fastener 300 includes a cap 330 and a double helix coil 312 having an increased retentive strength as well as means to balance surgical fastener 300 as it is pressed into tissue. A distal end potion 322 of double helix coil 312 terminates at two tips 324a, 324b for piercing and insertion into mesh 50 and tissue. Moreover, tips 324a, 324b are diametrically opposed to balance surgical fastener 300 as it is pressed into tissue.

Cap 330 includes a drive thread 334 configured to engage the inner thread of outer tube 23 of applicator 20. Cap 330 is, e.g., over-molded, onto a proximal end portion (not shown) of double helix coil 312, such that the proximal end portion of double helix coil 312 is enclosed by cap 330. Cap 330 has a larger diameter than a diameter of double helix coil 312 such that drive thread 334 engages the inner thread of outer tube 23, while double helix coil 312 is free of contact with the inner surface of outer tube 23 of applicator 20. Cap 330 includes a planar proximal surface 320 configured to reduce visceral attachment and adhesion. As with surgical fastener 100, the configuration of surgical fastener 300, e.g., the pre-formed pitch and diameter, may be varied for different applications and a barb (not shown) may be employed to enhance anchoring in tissue. Moreover, the materials contemplated are the same as those for helical fastener 100. Further, double helix coil 312 may also be longitudinally collapsible and expandable.

With reference now to FIG. 12, there is provided a surgical fastener 400 in accordance with yet another embodiment of the present disclosure. Surgical fastener 400 is substantially identical to surgical fastener 100. However, surgical fastener 400 includes a cap 430 that is tapered to facilitate attachment of fastener 400 against mesh 50 and tissue. Surgical fastener 400 also includes helical coil 412 extending distally from cap 430. Operation of surgical fasteners 300, 400 is substantially identical to the operation of surgical fastener 100 and thus will not be described herein.

Although the illustrative embodiments of the present disclosure have been described herein with reference to the accompanying drawings, the above description, disclosure, and figures should not be construed as limiting, but merely as exemplifications of particular embodiments. For example, while bore 132 is shown as having a D-shaped cross-section, it is also contemplated that bore 132 may include a square-shaped cross-section, as well as, a polygonal shaped and various other bore cross-sections, such as, e.g., oval or star shaped. Any non-circular shape for the cross-section of the bore is contemplated herein.

It is also to be appreciated that the present disclosure may be utilized in a number of applications including ligating tissue, hernia mesh repair, bladder neck suspension, and in conjunction with implant drug delivery systems or procedures involving positioning of surgical or implantable devices in patients. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A surgical fastener for attaching a prosthesis to tissue comprising:
   a cap defining a proximal surface and a bore; and
   a coil body extending distally from the cap, the coil body defining a channel configured to receive a drive rod of a surgical applicator inserted through the bore of the cap.
2. The surgical fastener according to paragraph 1, wherein the bore of the cap has a non-circular cross-section.
3. The surgical fastener according to paragraph 2, wherein the bore includes a planar portion and an arcuate portion.
4. The surgical fastener according to paragraph 1, wherein the coil body is a continuous double helical coil having a distal end and a proximal end, the distal end terminating with dual tips, each tip configured to pierce through tissue.
5. The surgical fastener according to paragraph 1, wherein the cap is over-molded onto a portion of the coil body.
6. The surgical fastener according to paragraph 5, wherein the cap is over-molded onto a proximal portion of the coil body.
7. The surgical fastener according to paragraph 1, wherein a diameter of the cap is larger than a diameter of the coil body.
8. The surgical fastener according to paragraph 1, wherein the coil body has a uniform diameter along a length thereof.
9. The surgical fastener according to paragraph 1, wherein the cap includes a material known to reduce visceral adhesions
10. The surgical fastener according to paragraph 9, wherein the material is at least one of glycerol or collagen.
11. The surgical fastener according to paragraph 1, wherein the cap is formed of a biologic material.
12. The surgical fastener according to paragraph 1, wherein the cap is formed of polymer or copolymer material.
13. The surgical fastener according to paragraph 12, wherein the cap is formed of absorbable, non-absorbable, or partially absorbable polymer or copolymer material.
14. The surgical fastener according to paragraph 1, wherein the coil body is longitudinally collapsible and expandable.
15. The surgical fastener according to paragraph 1, wherein the cap has a drive thread formed on a peripheral portion thereof.
16. The surgical fastener according to paragraph 15, wherein a leading edge of the drive thread is spaced apart from a proximal-most coil of the coil body to define a discontinuity therebetween.
17. The surgical fastener according to paragraph 15, wherein a trailing edge of the drive thread includes a proximal surface disposed flush with the proximal surface of the cap.
18. The surgical fastener according to paragraph 1, wherein the coil body is formed from a biocompatible metal.
19. The surgical fastener according to paragraph 1, wherein the coil body includes a tip configured to penetrate tissue as the surgical fastener is rotated relative to tissue.

## Claims

1. A surgical fastener for attaching a prosthesis to tissue comprising:
a cap defining a proximal surface and a bore; and
a coil body extending distally from the cap, the coil body defining a channel configured to receive a drive rod of a surgical applicator inserted through the bore of the cap.

2. The surgical fastener according to claim 1, wherein the bore of the cap has a non-circular cross-section; preferably wherein the bore includes a planar portion and an arcuate portion.

3. The surgical fastener according to claim 1 or claim 2, wherein the coil body is a continuous double helical coil having a distal end and a proximal end, the distal end terminating with dual tips, each tip configured to pierce through tissue.

4. The surgical fastener according to any preceding claim, wherein the cap is over-molded onto a portion of the coil body; preferably wherein the cap is over-molded onto a proximal portion of the coil body.

5. The surgical fastener according to any preceding claim, wherein a diameter of the cap is larger than a diameter of the coil body; and/or wherein the coil body has a uniform diameter along a length thereof.

6. The surgical fastener according to any preceding claim, wherein the cap includes a material known to reduce visceral adhesions; preferably wherein the material is at least one of glycerol or collagen.

7. The surgical fastener according to any preceding claim, wherein the cap is formed of a biologic material.

8. The surgical fastener according to any preceding claim, wherein the cap is formed of polymer or copolymer material.

9. The surgical fastener according to claim 8, wherein the cap is formed of absorbable, non-absorbable, or partially absorbable polymer or copolymer material.

10. The surgical fastener according to any preceding claim, wherein the coil body is longitudinally collapsible and expandable.

11. The surgical fastener according to any preceding claim, wherein the cap has a drive thread formed on a peripheral portion thereof.

12. The surgical fastener according to claim 11, wherein a leading edge of the drive thread is spaced apart from a proximal-most coil of the coil body to define a discontinuity therebetween.

13. The surgical fastener according to claim 12, wherein a trailing edge of the drive thread includes a proximal surface disposed flush with the proximal surface of the cap.

14. The surgical fastener according to any preceding claim, wherein the coil body is formed from a biocompatible metal.

15. The surgical fastener according to any preceding claim, wherein the coil body includes a tip configured to penetrate tissue as the surgical fastener is rotated relative to tissue.
